# EUROPEAN PATENT SPECIFICATION

(11) **EP 3 638 190 B1**
(45) Date of publication and mention of the grant of the patent: **28.08.2024**
(21) Application number: 18725313.3
(22) Date of filing: 17.04.2018
(51) Int. Cl.: A61K 8/26, A61K 8/73, A61K 8/02, A61Q 19/00, A61K 8/81, A61K 8/91, B32B 15/00, A45D 44/00, B32B 3/26, B32B 5/02, B32B 5/08, B32B 15/14, B32B 7/12, B32B 15/20, B32B 27/12

(54) **GEL SHEET MASK WITH METALLIC OCCLUSIVE LAYER**
GELFOLIENMASKE MIT METALLISCHER OKKLUSIONSSCHICHT
MASQUE DE FEUILLE DE GEL AYANT UNE COUCHE D'OCCLUSION MÉTALLIQUE

(30) Priority: 05.06.2017 JP 2017110772
(43) Date of publication of application: 22.04.2020
(73) Proprietor: L'OREAL, 75008 Paris (FR); Agarwal, Gaurav, Kawasaki-shi, Kanagawa, 213-0012 (JP); Ogata, Hiroyuki, Kawasaki-shi, Kanagawa, 213-0012 (JP); Ke, Lixuan, Nansha District 510000, Guangzhou (CN)
(72) Inventor: AGARWAL, Gaurav, Kawasaki-shi Kanagawa, 213-0012 (JP); OGATA, Hiroyuki, Kawasaki-shi Kanagawa, 213-0012 (JP); KE, Lixuan, 510000, Guangzhou (CN)
(74) Representative: Cabinet Nony
(86) International application number: PCT/JP2018/016381
(87) International publication number: WO 2018/225405

(56) References cited:
- EP-A1- 2 210 583
- CN-Y- 201 329 126
- FR-A1- 2 443 836
- JP-A- 2012 051 847
- DATABASE GNPD [online] MINTEL; 1 July 2016 (2016-07-01), THE OOZOO: "Face Golden Foil Mask", XP002782351, Database accession no. 4042357
- DATABASE GNPD [online] MINTEL; 1 January 2017 (2017-01-01), CARVER KOREA: "Premium Hydra Gold Foil Mask", XP002782352, Database accession no. 4562773
- DATABASE GNPD [online] MINTEL; 1 February 2017 (2017-02-01), NOHJ: "Golden Modelling Foil Mask", XP002782353, Database accession no. 4636553
- DATABASE GNPD [online] MINTEL; 1 July 2016 (2016-07-01), LG HOUSEHOLD & HEALTH CARE: "Golden Therapy Mask", XP002782354, Database accession no. 4150639
- DATABASE WPI Week 200831, 1 August 2008 Derwent World Patents Index; AN 2008-E56075, XP002782355
- DATABASE WPI Week 201222, 1 May 2012 Derwent World Patents Index; AN 2012-D14932, XP002782356

## Description

### [TECHNICAL FIELD]

The present invention relates to a gel sheet mask, in particular a facial gel sheet mask for skin care, with a metallic occlusive layer, as defined in the appended claims.

### [BACKGROUND ART]

A skin care mask which is a cosmetic product in the form of a sheet is well known. Such a sheet mask can be broadly divided into three categories as follows depending on the materials used for making the sheet:
1. Nonwoven sheet mask in which a fibrous sheet is impregnated with a cosmetic formulation;
2. Bio cellulose sheet mask in which a cellulosic sheet material is made by a microbial culture; and
3. Hydrogel sheet mask in which a sheet is made up of a water swollen network of cross linked polymers.

The nonwoven sheet mask is the most common product due to its low cost. On the other hand, the sensation on the skin, the capability for delivering a cosmetic composition to the skin, and the adhesion to the skin often depend on the type of nonwoven and the compatibility between the sheet material and the composition used.

The bio cellulose sheet mask offers a very favorable sensation because the sheet itself contains a high amount of water and it also exhibits good adhesion to the skin. However, such a sheet mask is very expensive due to its production process with low yield.

From a cost perspective, the hydrogel sheet mask can be positioned in between the nonwoven sheet mask and the bio cellulose sheet mask. Due to the very high water content in the sheet, this kind of sheet mask is known to be excellent with respect to its transparency, good adherence to the skin, and hydration properties.

Although the hydrogel sheet is made up of a 3-D structure of polymers within a water medium, from a manufacturing perspective, it still needs a liner to cast the hydrogel formulation and also needs another protection liner to cover the casted hydrogel sheet. Therefore, in the case that this hydrogel sheet is impregnated with a cosmetic composition, a portion of the cosmetic composition will adhere to these liners while another portion of the cosmetic composition will drip in the sachet.

Second, since the hydrogel sheet mask is composed of a high amount of water and other hydrophilic ingredients, good immediate hydration benefits are obtained but a long-lasting benefit of hydration and other cosmetic benefits are lost.

Another limitation of the hydrogel production process is that the polymers to be used need to be jellified at high temperature and need to be cooled down after casting to form a gel sheet, and this heating-cooling process makes hydrogel sheets expensive.

Depending on the composition of the hydrogel, in order to provide enough mechanical strength to the mask sheet, the hydrogel composition may also be casted around a thin sheet of mesh or nonwoven.

Prior art related to the present invention is disclosed in the following publications.

WO 2008/072817 A1 proposes a method for manufacturing a hydrogel sheet by filling the composition in a frame and then submerging it in a metal salt solution. This procedure may avoid the use of thin sheet materials. However, the process of filling the composition into an individual mold for making a mask still has low yield.

In order to improve the effectiveness of the hydrogel mask for delivering more benefits to skin, Korean Patent Publication No. 100864541 proposes a phase change hydrogel composition. According to this patent, during use, due to skin temperature, the hydrogel composition can be more liquefied and therefore, more composition can be delivered to the skin.

CN 100864541 proposes a cream coated hydrogel mask. More particularly, relating to a stable emulsion of a hydrogel surface, a multilayer structure is coated with a thin layer of water in the cream gel mask.

Korean Patent Publication No. 101372227 B1 describes a hydrogel mask coated with a lipophilic film and sprayed with a composition for curing of the film. During the use of such a face mask, a cured surface is provided as a support, and the other side which is not cured is provided for attachment to the face for beauty care.

The prior art mentioned above provides a solution for a hydrogel mask with more cosmetic benefits. However, they use the conventional process of making a hydrogel and a further process of adding a cream, special ingredients, or a lipophilic film, which further increases the cost of the product.

JP 5719130 proposes a two-layer sheet mask having a formula between these two layers. The two-layer sheet comprises a first layer which is made of various fiber materials and a second layer made of a polymer-based film. The formula must be an O/W (oil-in-water) emulsion. Such a two-layer sheet mask has limited occlusive properties and during the application of the mask, the edges of the mask may start to dry out. In particular, if only aqueous gels are used, this drying problem at the edges becomes even more severe.

One may also cite face masks formulated with gold as mentioned for example in the documents: DATABASE GNPD [Online] MINTEL; 1 July 2016 (2016-07-01), The Oozoo: "Face Golden Foil Mask", XP002782351, Database accession no. 4042357; DATABASE GNPD [Online] MINTEL; 1 January 2017 (2017-01-01), Carver Korea: "Premium Hydra Gold Foil Mask", XP002782352, Database accession no. 456277; DATABASE GNPD [Online] MINTEL; 1 February 2017 (20147-02-01), NOHJ: "Golden Modelling Foil Mask", XP002782353, Database accession no. 4636553 and DATABASE GNPD [Online] MINTEL; 1 July 2016 (2016-07-01), LG Household & Health Care: "Golden Therapy Mask", XP002782354, Database accession no. 4150639, CN 201329126 and JP3139140.

JP 2012051847 relates to a patch comprising a preparation for skin sandwiched between two external supports.

FR 2 443 836 relates to a method for treating the skin in which the area of the body is covered,a feter having coated it with an active product, with a metallic film.

EP 2 210 583 relates to a cosmetic preparation comprising a gel sheet comprising a hydrogel.

### [DISCLOSURE OF THE INVENTION]

In view of the above, an object of the present invention is to provide a novel gel sheet mask which can overcome the limitations of the prior art sheet masks. In particular, an object of the present invention is to provide a novel gel sheet mask which can overcome the fitting problems of the prior art sheet masks. Another object of the present invention is to provide a novel gel sheet mask which can enhance the experience derived from its usage, in particular, a novel gel sheet mask which can provide a warming effect to a user.

In order to achieve the above-described objects, the present invention provides a gel sheet mask including a sheet-like support layer and a gel layer that consists of a gel composition and that is applied to one surface of the support layer, wherein the gel sheet mask further includes a metallic occlusive layer that is applied to the other surface of the support layer, which is different from the surface of the support layer to which the gel layer is applied, so as to cover the other surface of the support layer. Owing to the fact that the metallic occlusive layer more completely follows the contours of a user's face compared to a simple polymer-based film, the gel sheet mask with the above-described constitution can be easily adhered to the user's face in a non-messy manner with good adhesion to the skin. In addition, the gel sheet mask according to the present invention can further enhance, for example, the cosmetic benefits of the gel composition due to the existence of the metallic occlusive layer. Furthermore, due to the existence of the metallic occlusive layer, the gel sheet mask according to the present invention can enhance the experience derived from the use thereof, and in particular can provide a warming sensation to the user. This is believed to be mainly because (i) the volatilization of the gel composition which causes a drop in skin temperature is suppressed due to the existence of the metallic occlusive layer, and (ii) the metallic occlusive layer returns heat that was released from the user's face back to the user's face due to the heat radiation reflection effect thereof.

The term "mask" as used in the present specification is intended to include not only masks that cover the entirety of a user's face, but also, for example, half masks that cover only the upper half or lower half of a user's face. Further, for example, facial patches are also included in the term "mask" as used in the present specification.

According to a preferred aspect of the present invention, the gel sheet mask has a center line, and during non-use, such as during storage or transport, the gel sheet mask is in a state of being folded in half centered on the center line so that the gel layer is sandwiched by the support layer. The gel sheet mask which has been folded in half is unfolded by a user immediately before use.

According to a preferred aspect of the present invention, the metallic occlusive layer may consist of a foil made of one material selected from the group consisting of gold, silver, and aluminum. Alternatively, the metallic occlusive layer may be a plastic film, wherein one material selected from the group consisting of gold, silver, and aluminum is deposited on at least one surface of the plastic film. Further, the metallic occlusive layer may be a layer of one material selected from the group consisting of gold, silver, and aluminum that is directly deposited onto the other surface of the support layer.

According to a preferred aspect of the present invention, the support layer may be capable of absorbing at least a portion of the gel composition that is applied thereto. Further, the support layer may be hydrophilic. In particular, the support layer may consist of a nonwoven sheet made from cellulose.

According to a preferred aspect of the present invention, the gel composition may be selected from the group consisting of an aqueous gel, an oil-in-water gel, a water-in-oil gel, and a water-in-silicone gel. Further, the gel composition may include a polymer, and the polymer may be selected from the group consisting of carbomer, xanthan gum, cellulose, polysaccharides, polyacrylate, polyethylene, and polyester graft polyacrylate copolymers.

According to a preferred aspect of the present invention, the coating amount of the gel composition may be 50 to 2000 grams per square meter, preferably 400 to 1000 grams per square meter. Further, the viscosity of the gel composition may be 6000 centipoise or more.

### [BRIEF DESCRIPTION OF THE DRAWINGS]

Non-limiting and representative embodiments of the present invention will now be explained in detail below referring to the attached drawings.
FIG. 1 is a plan view of a gel sheet mask according to one embodiment of the present invention, wherein the gel sheet mask is in a spread-out state.
FIG. 2 is a cross-section view of the gel sheet mask along line Y-Y in FIG. 1.
FIG. 3 is a perspective view illustrating the sheet mask of FIG. 1 in a state in which the sheet mask is folded in half centered on a center line X.
FIG. 4 is a cross-section view of the folded gel sheet mask along line Z-Z in FIG. 2.
FIG. 5 is a diagram illustrating one example of a system used for manufacturing the gel sheet mask in a state in which the gel sheet mask is folded in half as shown in FIG. 3.
FIG. 6 is a graph illustrating the weight of gel composition that has dripped into the sachet from a sample of a mask accommodated in a sachet (Example 1).
FIG. 7 is a graph illustrating the weight of gel composition transferred onto the face of a tester from a sample of a mask adhered to the face of the tester (Example 1).
FIG. 8 is a graph illustrating the results from measuring the skin hydration value (Example 1).
FIG. 9 is a graph illustrating the results from measuring the skin TEWL (Transepidermal Water Loss) value (Example 1).
FIG. 10 is a graph illustrating a temperature change of skin on the arm of a tester upon adhering a sample of a patch (Example 2).

### [DETAILED DESCRIPTION OF EMBODIMENTS]

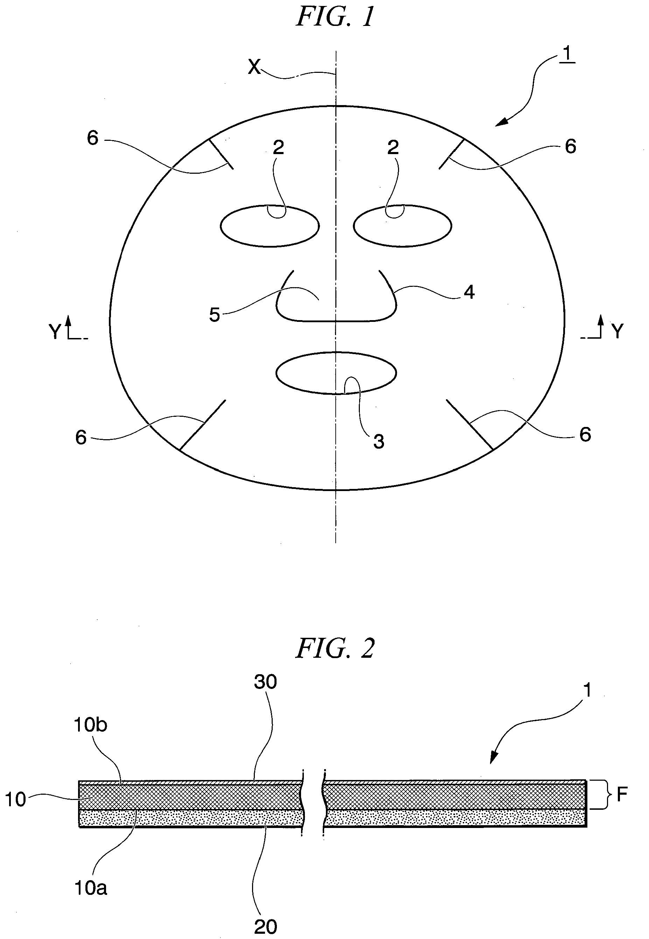

An embodiment of the present invention will now be explained referring to FIGS. 1 to 4. The following embodiment is a sheet mask which can cover the entire face of a user. However, the present invention can be similarly applied to sheet masks of other forms as well.

FIG. 1 illustrates a gel sheet mask 1 according to an embodiment of the present invention in a spread-out state, i.e. in a state immediately before use. The mask 1 has a center axis line X, which is a left-right symmetrical axis line that divides the mask 1 into two equal parts. In the mask 1, elliptical openings 2 and 3 are formed in positions corresponding to the eyes and mouth of the user. A U-shaped slit 4 is provided at a position corresponding to the nose of the user of the mask 1, and by means of this slit 4, a flap 5 is formed in the mask 1. When a user adheres the mask 1 to his or her face, the user's nose can be made to protrude from the remaining surface of the mask 1 due to the existence of the flap 5. Further, in order to enhance the fit on the user's face, slits 6 along the radial direction are formed at, for example, four places on the peripheral edge of the mask 1.

As can be understood from FIG. 2, which shows a cross-section of the gel sheet mask 1 along line Y-Y in FIG. 1, the mask 1 includes the following: a sheet-like support layer 10; a gel layer 20 that consists of a gel composition, particularly a cosmetic composition, and that is applied to one surface 10a of the support layer 10; and a metallic occlusive layer 30 that is applied to the other surface 10b of the support layer 10, which opposes the surface 10a, so as to cover the other surface 10b. In FIG. 2, it should be understood that the thickness of each layer is exaggerated.

In the present embodiment, the metallic occlusive layer 30 consists of a foil made of aluminum, and the metallic occlusive layer 30 is joined using a suitable adhesive to the surface 10b of the support layer 10. However, a foil made of a metallic material other than aluminum such as gold or silver can be used as the metallic occlusive layer 30. The metallic occlusive layer 30 functions to prevent the gel composition which has been coated on the surface 10a of the support layer 10 from passing through the support layer 10 and leaking from the opposite surface 10b. In other words, the surface 10b of the support layer 10 is occluded due to the existence of the metallic occlusive layer 30.

In another embodiment, the metallic occlusive layer 30 may be made of a plastic film, wherein a metallic material such as gold, silver, or aluminum is deposited on at least one surface of the plastic film. In this case, the plastic film on which the metallic material is deposited may be joined using a suitable adhesive to the surface 10b of the support layer 10.

In yet another embodiment, the metallic occlusive layer 30 may be a layer of a metallic material such as gold, silver, or aluminum that is directly deposited onto the surface 10b of the support layer 10.

The support layer 10 functions to support both the gel layer 20 and the metallic occlusive layer 30. In the present embodiment, as the support layer 10, for example, a layer manufactured from a nonwoven sheet made from natural or artificial cellulose fibers, and more specifically a layer manufactured from a viscose spun lace nonwoven sheet, is used. Thus, the support layer 10 is hydrophilic, and is capable of absorbing at least a portion of the gel composition that is applied to the surface 10a. The support layer 10 forms an occlusive sheet F in cooperation with the metallic occlusive layer 30.

In the present embodiment, as the gel composition which forms the gel layer 20, an aqueous gel, an oil-in-water (O/W) gel, a water-in-oil (W/O) gel, or a water-in-silicone (W/Si) gel cream is used. In particular, the gel composition may include a polymer selected from carbomer, xanthan gum, cellulose, polysaccharides, polyacrylate, polyethylene, and polyester graft polyacrylate copolymers. The coating amount of the gel composition is 50 to 2000 grams/m² (gsm), and preferably 400 to 1000 grams/m² (gsm). Further, the viscosity of the gel composition is 10000 centipoise or more.

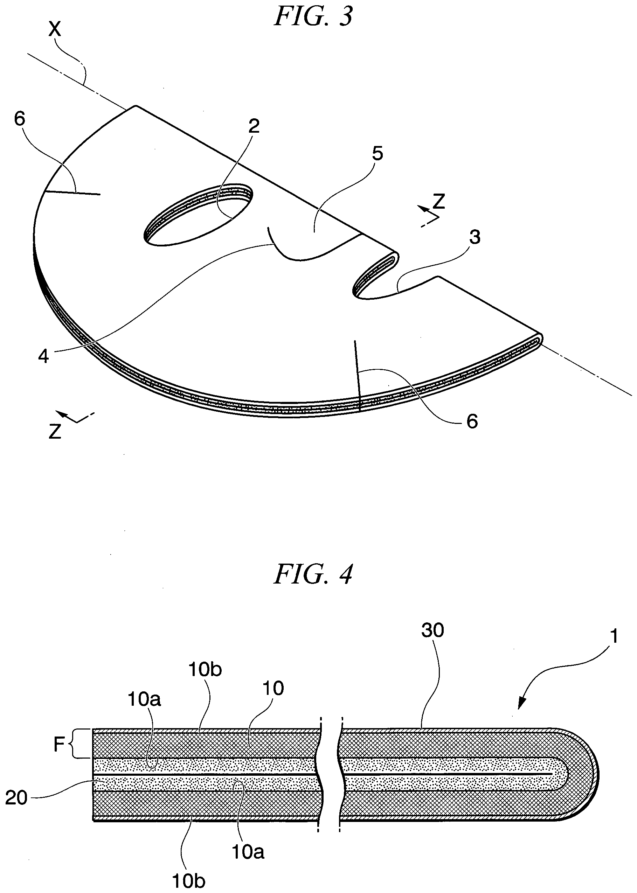

FIG. 3 illustrates the gel sheet mask 1 of FIG. 1 in a state in which the sheet mask is folded in half centered on the center line X. As explained below, the gel sheet mask 1 is in the state shown in FIG. 3 upon completion of the manufacture thereof. As can be understood from FIG. 4, which shows a cross-section of the gel sheet mask 1 along line Z-Z in FIG. 3, until immediately before use, the gel sheet mask 1 is maintained in a state in which it is folded in half centered on the center line X so that the gel layer 20 is sandwiched by the support layer 10, or more accurately by the occlusive sheet F. In this folded state, the gel sheet mask 1 is accommodated in a sachet, tray, or some other package (not illustrated), and then is transported and stored. In order to save space, the gel sheet mask 1 may be further folded in half, i.e. folded to 1/4 its original size. In FIG. 4, it should be understood that the thickness of each layer is exaggerated.

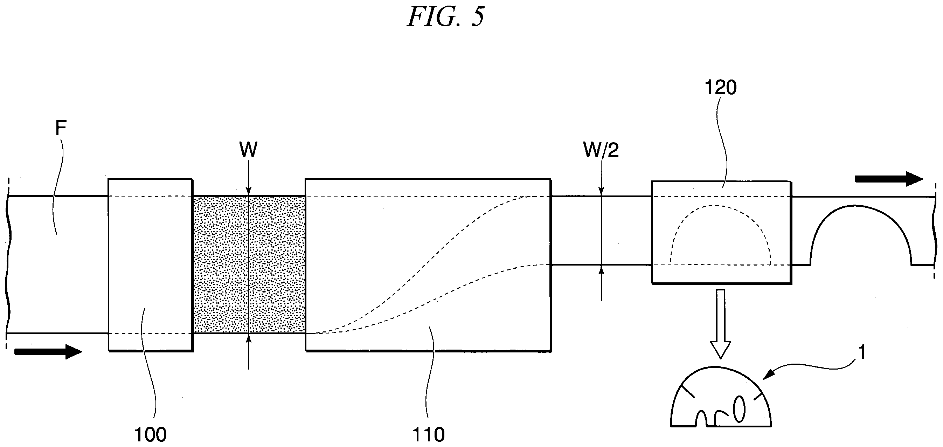

An example of a method for manufacturing the gel sheet mask will now be roughly explained below. In the manufacture of the gel sheet mask, the system shown in FIG. 5 can be used. This system includes the following from the upstream side to the downstream side: (i) a supply source (not illustrated) of the occlusive sheet F that is formed by the support layer 10 and the metallic occlusive layer 30; (ii) a coating unit, particularly a knife coater 100; (iii) a folding unit 110; and (iv) a punching unit 120. During operation of this system, first, the occlusive sheet F is supplied to the knife coater 100 with the support layer side facing upwards. The supply direction of the occlusive sheet F is parallel to the center line of the mask to be ultimately obtained. In the knife coater 100, the gel composition is uniformly coated onto the top surface of the occlusive sheet F (the surface of the support layer). Next, the occlusive sheet F onto which the gel composition has been coated is supplied to the folding unit 110, where the occlusive sheet F is folded in half so that the gel composition is sandwiched by the occlusive sheet F. As a result, the width of the occlusive sheet F onto which the gel composition has been coated becomes half (W/2) of its width W before being supplied to the folding unit 110. Next, the occlusive sheet F onto which the gel composition has been coated and which has been folded in half is supplied to the punching unit 120, where the occlusive sheet F is punched to an approximately semicircular shape using a die (not illustrated). At the same time, the openings corresponding to the eyes and mouth of the user and the slit which defines the nose flap are formed in the occlusive sheet F which has been punched to a semicircular shape. As a result, the gel sheet mask 1 which is in the state of being folded in half as shown in FIG. 3 and which is in the state where the gel composition is sandwiched between the two adjacent semicircular regions of the occlusive sheet F is obtained.

### Example 1: Hydration and TEWL Effects of the Gel Sheet Mask

As the support layer, a sheet of nonwoven material consisting of 95 wt% cellulosic fibers and 5 wt% polyethylene fibers was prepared. Onto one surface of this sheet, an aluminum foil having a thickness of 7 µm was adhered as the metallic occlusive layer, and thereby an occlusive sheet was formed. Further, two gel compositions, i.e. Composition A and Composition B, having the proportions of ingredients shown in Table 1 were prepared. Composition A is an aqueous gel, whereas Composition B is an O/W emulsion. Compositions A and B were coated onto one surface of separate occlusive sheets. For both compositions, the coating amount and the coating amount per unit area were 16.38 grams and 540 gsm (grams per square meter). In this way, two samples of the gel sheet mask explained above using FIGS. 1 to 4 respectively including Composition A or Composition B were produced. Hereinafter, these samples will be referred to as Sample A and Sample B.

**Table 1: Proportion of Ingredients of Compositions A and B**

| Ingredient | Composition A (wt%) | Composition B (wt%) |
|---|---|---|
| Water | 93.35 | 84.418 |
| Glycerin | 5 | 6 |
| Xanthan Gum | 0.1 | 0 |
| Carbomer | 0.5 | 0 |
| Biosaccharide Gum | 1 | 0 |
| Sodium Hydroxide | 0.05 | 0 |
| Butylene Glycol | 0 | 2 |
| Di propylene Glycol | 0 | 3 |
| PEG:8 | 0 | 1 |
| Dimethicone | 0 | 2.5 |
| Carbomer | 0 | 0.3 |
| Polyacryloyldimethyl Taurate | 0 | 0.75 |
| Sodium Hydroxide | 0 | 0.032 |

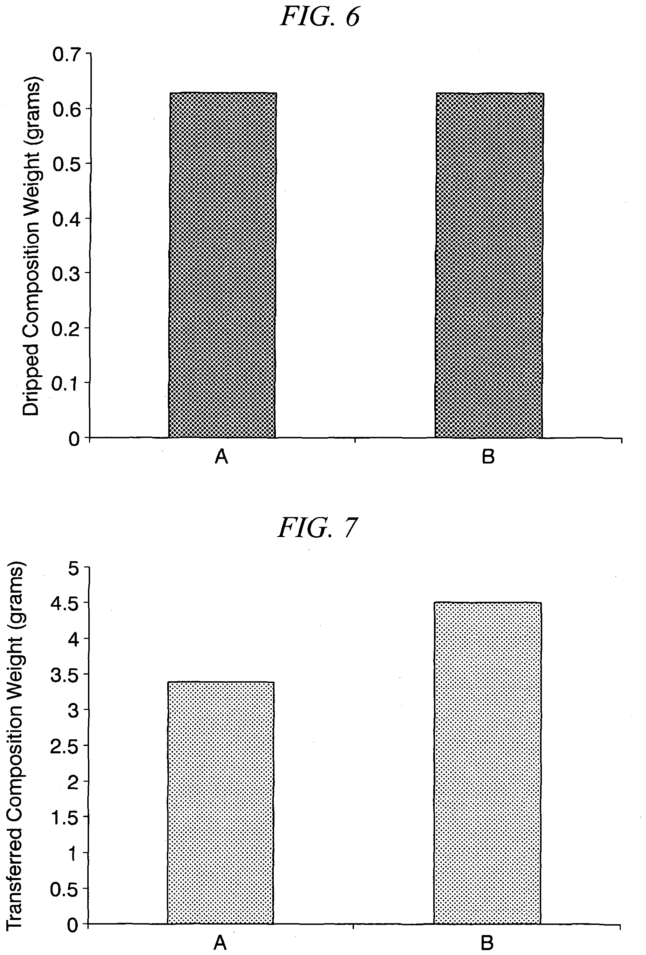

Samples A and B produced as described above were then accommodated in aluminum sachets and retained in this state for 7 days. After 7 days, the sachets were opened, and the weight of the gel composition that had dripped into the sachets was calculated. The results thereof are shown in the graph of FIG. 6.

Next, Samples A and B were adhered to the face of a tester. After 15 minutes, Samples A and B were removed from the tester's face, and the weight of the gel composition which remained on the face, i.e. which transferred to the skin, was calculated. The results thereof are shown in the graph of FIG. 7.

From the graph of FIG. 6, it can be understood that the weight of the gel composition that had dripped into the sachets was only 3 to 5% of the weight of the originally coated gel composition. Meanwhile, from the graph of FIG. 7, it can be understood that, for Sample A, the weight of the gel composition remaining on the face reached about 20% of the weight of the originally coated gel composition, whereas for Sample B, the weight of the gel composition remaining on the face reached about 26% of the weight of the originally coated gel composition.

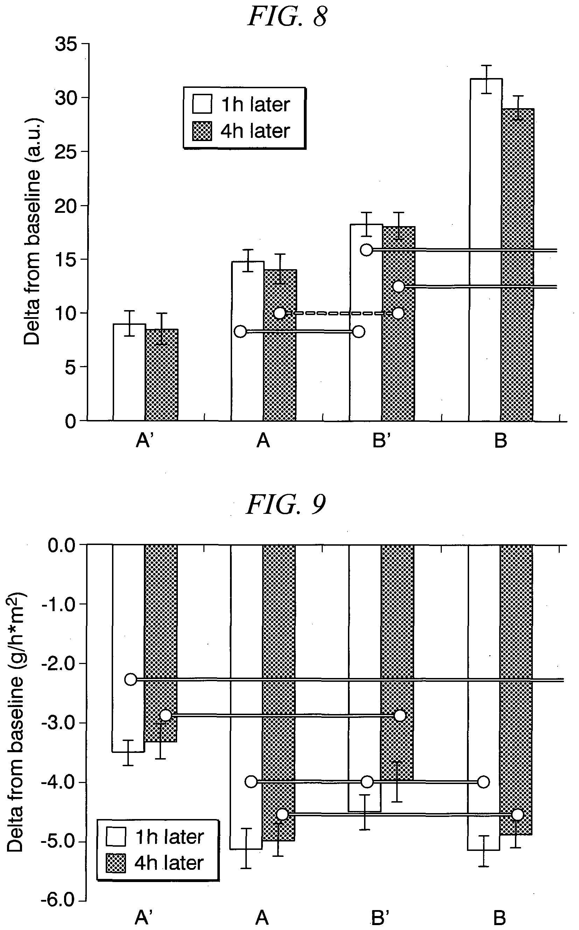

Next, Samples A and B were cut to 5 cm x 5 cm to prepare two patches. Hereinafter, these patches will be referred to as Patch A and Patch B. Patches A and B were then adhered to the forearm of a tester for 15 minutes. The hydration value and the TEWL (Transepidermal Water Loss) value of the skin were measured 1 hour and 4 hours after adhering Patches A and B. A Corneometer0 was used for measuring the hydration value. The measurement results of the hydration value are shown in FIG. 8, and the measurement results of the TEWL value are shown in FIG. 9. In the graphs of FIGS. 8 and 9, the measurement results after 1 hour and the measurement results after 4 hours are illustrated with two adjacent bars. The left bar is the measurement results after 1 hour, and the right bar is the measurement results after 4 hours. For comparison, the hydration and TEWL values of the skin were also measured upon simply coating Compositions A and B on the forearm. The coating amount was 0.16 grams for both Composition A and Composition B. The measurement results for comparison are indicated by A' and B' in the graphs of FIGS. 8 and 9.

From the graph of FIG. 8, it can be understood that Patch A greatly increases the skin hydration compared to the mere application of Composition A. The same can be said about Patch B. It should be noted that the hydration effect achieved by Patch A and Patch B was maintained at a high level even after 4 hours had elapsed from adhering the patch. Meanwhile, from FIG. 9, it can be understood that the use of Patch A greatly enhances the skin barrier properties compared to the mere application of Composition A, and thus moisture loss from the skin can be greatly reduced. The same can be said about Patch B. Further, it should be noted that Patch A, to which the aqueous gel composition of Composition A was applied, exhibits barrier properties equivalent to those of Patch B, to which the O/W emulsion of Composition B was applied.

### Example 2: Warming Effect of Gel Sheet Mask

In order to confirm the warming effect achieved by the gel sheet mask of the present invention, four types of sheet masks P, Q, R, and S with mutually different constitutions were prepared.

The mask sheet P (Comparative Example 1) includes a one-layer sheet consisting of cellulosic nonwoven made of viscose and pulp. Composition A used in Example 1 was coated onto one surface of the sheet. The mask sheet Q (Comparative Example 2) includes a two-layer sheet consisting of a first layer of cellulosic nonwoven and a second layer of PE film. The thickness of the PE film was 5 µm. Composition A used in Example 1 was coated onto the surface of the first layer on the opposite side of the surface to which the PE film was applied. The mask sheet R (Example 1 of the Present Invention) includes two-layer sheet consisting of a first layer of cellulosic material and a second layer of aluminum foil. The thickness of the aluminum foil was 7 µm. Composition A used in Example 1 was coated onto the surface of the first layer on the opposite side of the surface to which the aluminum foil was applied. The mask sheet S (Example 2 of the Present Invention) includes a two-layer sheet consisting of a first layer of cellulosic material and a second layer of PE film onto which aluminum has been vapor deposited. The thickness of the PE film was 5 µm. Composition A used in Example 1 was coated onto the surface of the first layer on the opposite side of the surface onto which the PE film was applied.

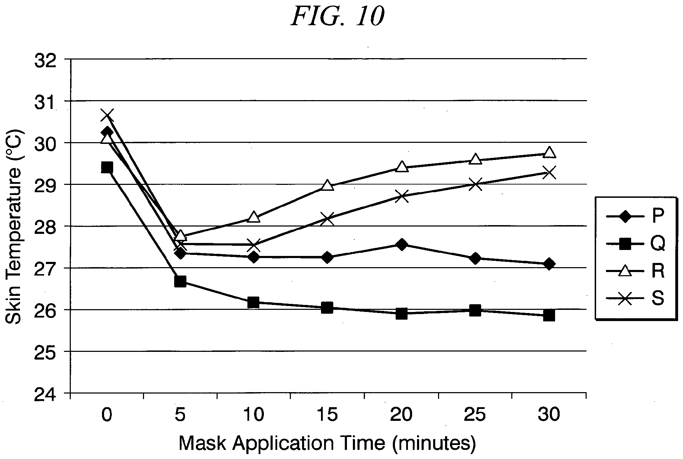

These four mask sheets P, Q, R, and S were adhered to the forearm of a tester. Next, the skin temperature under the mask sheets P, Q, R, and S was continuously measured for 30 minutes. The results thereof are shown in the graph of FIG. 10.

As is clear from FIG. 10, with all of the four mask sheets P, Q, R, and S, the skin temperature began to drop immediately after the start of the test, and then the skin temperature and the sheet mask temperature reached equilibrium after about 5 minutes. In the case of the mask sheets P and Q which do not include a metallic occlusive layer, the skin temperature remained almost constant after reaching the temperature equilibrium. On the other hand, in the case of the mask sheets R and S which include a metallic occlusive layer, the skin temperature began to rise after reaching the temperature equilibrium. Therefore, it can be understood that the mask sheets R and S can provide a warming sensation to the tester (and thus the mask user) after about 5 minutes has elapsed from adhering the mask sheet.

## Claims

1. A gel sheet mask (1), comprising:
a sheet-like support layer (10); and
a gel layer (20) that consists of a gel composition and that is applied to one surface (10a) of the support layer (10),
wherein the gel sheet mask (1) further comprises a metallic occlusive layer (30) that is applied to the other surface (10b) of the support layer (10), which is different from the surface (10a) of the support layer (10) to which the gel layer (20) is applied, so as to cover the other surface (10b) of the support layer (10);
wherein the gel sheet mask (1) has a center line (X), and during non-use, the gel sheet mask (1) is in a state of being folded in half centered on the center line (X) so that the gel layer (20) is sandwiched by the support layer (10);
wherein the metallic occlusive layer (30) is a layer of one material selected from the group consisting of gold, silver, and aluminum that is directly adhered onto the other surface (10b) of the support layer (10).

2. The gel sheet mask (1) according to claim 1, wherein the support layer (10) consists of a nonwoven sheet made from cellulose.

3. The gel sheet mask (1) according to claim 1 or claim 2, wherein the gel composition is selected from the group consisting of an aqueous gel, an oil-in-water gel, a water-in-oil gel, and a water-in-silicone gel.

4. The gel sheet mask (1) according to any one of claims 1 to 3, wherein the gel composition comprises a polymer, and the polymer is selected from the group consisting of carbomer, xanthan gum, cellulose, polysaccharides, polyacrylate, polyethylene, and polyester graft polyacrylate copolymers.

## Patentansprüche

1. Geltuchmaske (1), umfassend:
eine tuchartige Trägerschicht (10); und
eine Gelschicht (20), die aus einer Gelzusammensetzung besteht und die auf eine Oberfläche (10a) der Trägerschicht (10) aufgebracht ist,
wobei die Geltuchmaske (1) ferner eine metallische Okklusionsschicht (30) umfasst, die auf die andere Oberfläche (10b) der Trägerschicht (10), die von der Oberfläche (10a) der Trägerschicht (10), auf die die Gelschicht (20) aufgebracht ist, verschieden ist, aufgebracht ist, um die andere Oberfläche (10b) der Trägerschicht (10) zu bedecken;
wobei die Geltuchmaske (1) eine Mittellinie (X) aufweist und die Geltuchmaske (1) sich während der Nichtverwendung in einem Zustand befindet, in dem sie auf der Mittellinie (X) mittig zu Hälften gefaltet ist, sodass die Gelschicht (20) sandwichartig von der Trägerschicht (10) eingeschlossen ist;
wobei die metallische Okklusionsschicht (30) eine Schicht aus einem Material ist, das aus der Gruppe bestehend aus Gold, Silber und Aluminium ausgewählt ist und direkt auf die andere Oberfläche (10b) der Trägerschicht (10) geklebt ist.

2. Geltuchmaske (1) nach Anspruch 1, wobei die Trägerschicht (10) aus einem aus Cellulose hergestellten Vliestuch besteht.

3. Geltuchmaske (1) nach Anspruch 1 oder Anspruch 2, wobei die Gelzusammensetzung aus der Gruppe bestehend aus einem wässrigen Gel, einem Öl-in-Wasser-Gel, einem Wasser-in-Öl-Gel und einem Wasser-in-Silikon-Gel ausgewählt ist.

4. Geltuchmaske (1) nach einem der Ansprüche 1 bis 3, wobei die Gelzusammensetzung ein Polymer umfasst und das Polymer aus der Gruppe bestehend aus Carbomer, Xanthangummi, Cellulose, Polysacchariden, Polyacrylat, Polyethylen und Polyester-Pfropf-Polyacrylat-Copolymeren ausgewählt ist.

## Revendications

1. Masque de feuille de gel (1), comprenant :
une couche de support de type feuille (10) ; et
une couche de gel (20) qui est constituée d'une composition de gel et qui est appliquée sur une surface (10a) de la couche de support (10),
dans lequel le masque de feuille de gel (1) comprend en outre une couche occlusive métallique (30) qui est appliquée sur l'autre surface (10b) de la couche de support (10), qui est différente de la surface (10a) de la couche de support (10) sur laquelle la couche de gel (20) est appliquée, de manière à recouvrir l'autre surface (10b) de la couche de support (10) ;
dans lequel le masque de feuille de gel (1) a une ligne centrale (X), et en cas de non-utilisation, le masque de feuille de gel (1) est dans un état dans lequel il est plié en deux centré sur la ligne centrale (X) de sorte que la couche de gel (20) soit prise en sandwich par la couche de support (10) ;
dans lequel la couche occlusive métallique (30) est une couche d'un matériau sélectionné dans le groupe constitué d'or, d'argent et d'aluminium qui est directement collée sur l'autre surface (10b) de la couche de support (10).

2. Masque de feuille de gel (1) selon la revendication 1, dans lequel la couche de support (10) est constituée d'une feuille non tissée composée de cellulose.

3. Masque de feuille de gel (1) selon la revendication 1 ou la revendication 2, dans lequel la composition de gel est sélectionnée dans le groupe constitué d'un gel aqueux, d'un gel d'huile-dans-eau, d'un gel d'eau-dans-huile et d'un gel d'eau-dans-silicone.

4. Masque de feuille de gel (1) selon l'une quelconque des revendications 1 à 3, dans lequel la composition de gel comprend un polymère, et le polymère est sélectionné dans le groupe constitué d'un carbomère, d'une gomme xanthane, d'une cellulose, de polysaccharides, d'un polyacrylate, d'un polyéthylène et de copolymères de polyacrylate greffé par polyester.
